# EUROPEAN PATENT SPECIFICATION

(11) **EP 0 980 381 B1**
(45) Date of publication and mention of the grant of the patent: **06.08.2003**
(21) Application number: 98918786.9
(22) Date of filing: 28.04.1998
(51) Int. Cl.: C07J 31/00, A61K 31/57

(54) **PHARMACEUTICALLY ACCEPTABLE SALTS OF 5.ALPHA.-PREGN-16-EN-3.BETA.-OL-20-ONE 3-SULPHATE ESTER WITH PROGESTIN ACTIVITY AND USEFUL IN THE TREATMENT OF CNS DISORDERS**
PHARAMEZEUTISCHE ANWENDBARE SÄLZE VON 5.ALPHA.-PREGN-16-EN-3.BETA.-OL-20-ON 3-SULPHATE ESTER MIT GESTAGEN AKTIVITÄT UND VERWENDBAR ZUR BEHANDLUNG VON STÖRUNGEN DES ZNS
SELS DE 5.ALPHA.-PREGN-16-EN-3.BETA.-OL-20-ONE 3-SULFATE ESTER A ACTIVITE PROGESTATIVE POUR LE TRAITEMENT DES CONDITIONS LIEES AU SNC

(30) Priority: 02.05.1997 US 850569
(43) Date of publication of application: 23.02.2000
(73) Proprietor: Wyeth, Madison, New Jersey 07940-0874 (US)
(72) Inventor: BENDER, Reinhold, Hans, Wilhelm, Valley Forge, PA 19481 (US); FAWZI, Mahdi, Bakir, Morristown, NJ 07960 (US); FLETCHER, Horace, III, Pottstown, PA 19464 (US); MORTON, George, Oscar, Hillsdale, NJ 07642 (US); SHAH, Syed, Muzafar, East Hanover, NJ 07936 (US); TANG, Xuejun, New City, NY 10956 (US)
(74) Representative: Talbott, Dawn Jacqueline
(86) International application number: US9808453
(87) International publication number: WO98050410

(56) References cited:
- GYERMEK L ET AL: "Steroids. CCCX. Structure-activity relation of some steroidal hypnotic agents" JOURNAL OF MEDICINAL CHEMISTRY., vol. 11, no. 1, January 1968, WASHINGTON US, pages 117-125, XP002070988
- CHEMICAL ABSTRACTS, vol. 126, no. 3, 20 January 1997 Columbus, Ohio, US; abstract no. 026914, BOLGER M B ET AL: "In vitro and in vivo activity of 16,17-dehydro-epipregnanolones: 17,20-bond torsional energy analysis and D-ring conformation" XP002070989 & PHARM. RES. , vol. 13, no. 10, 1996, pages 1488-1494,
- PHILLIPPS G H: "STRUCTURE-ACTIVITY RELATIONSHIPS IN STEROIDAL ANAESTHETICS" JOURNAL OF STEROID BIOCHEMISTRY, vol. 6, no. 5, May 1975, pages 607-613, XP000600716
- IM W B ET AL: "STUDIES ON THE MECHANISM OF INTERACTIONS BETWEEN ANESTHETIC STEROIDS AND GAMMA-AMINOBUTYRIC ACIDA RECEPTORS" MOLECULAR PHARMACOLOGY, vol. 37, no. 3, March 1990, pages 429-434, XP000600697
- SMITH H E ET AL: "BINDING OF STEROIDS TO PROGESTERONE RECEPTOR PROTEINS IN CHICK OVIDUCT AND HUMAN UTERUS" JOURNAL OF BIOLOGICAL CHEMISTRY, vol. 249, no. 18, 25 September 1974, pages 5924-5932, XP000610861

## Description

### BACKGROUND OF THE INVENTION

The use of naturally occurring estrogenic compositions of substantial purity and low toxicity such as PREMARIN (conjugated equine estrogens) has become a preferred medical treatment for alleviating the symptoms of menopausal syndrome, osteoporosis/osteopenia in estrogen deficient women and in other hormone related disorders. The estrogenic components of the naturally occurring estrogenic compositions have been generally identified as sulfate esters of estrone, equilin, equilenin, 17-β-estradiol, dihydroequilenin and 17-β-dihydroequilenin (U.S. Patent 2,834,712). The estrogenic compositions are usually buffered or stabilized with alkali metal salts of organic or inorganic acids at a substantially neutral pH of about 6.5 to 7.5. Urea has also been used as a stabilizer (U.S. 3,608,077). The incorporation of antioxidants to stabilize synthetic conjugated estrogens and the failure of pH control with tris(hydroxymethyl)aminomethane (TRIS) to prevent hydrolysis is discussed in U.S. 4,154,820.

One of the compounds described herein, 5α-pregn-16-en-3β-ol-20-one 3-sulfate ester sodium salt is a minor component of PREMARIN (conjugated equine estrogens).

### DESCRIPTION OF THE INVENTION

"J. Med. Chem vol 11(1) pp117-125; Chemical Abstracts 126: 026,914; J. Steroid Biochem vol 6 pp 607-613; Mol. Pharmacol vol 37(3) pp 429-434 and J. Biol. Chem. vol 249(18) pp 5924-5932 disclose steroids, including some Δ¹⁶⁽¹⁷⁾ progesterone derivatives, with some CNS activity or progestational activity."

In accordance with this invention, there is provided a pharmaceutically acceptable salt of 5α-pregn-16-en-3β-ol-20-one 3-sulfate ester which is useful as a progestational agent. The structure of 5α-pregn-16-en-3β-ol-20-one is shown as compound **(1)** in Scheme I.

Pharmaceutically acceptable salts of 5α-pregn-16-en-3β-ol-20-one 3-sulfate ester include, but are not limited to, the alkali metal salts, alkaline earth metal salts, ammonium salts, alkylammonium salts containing 1-6 carbon atoms or dialkylammonium salts containing 1-6 carbon atoms in each alkyl group, trialkylammonium salts containing 1-6 carbon atoms in each alkyl group and tetraalkylammonium salts containing 1-6 carbon atoms in each alkyl group.

Alkali metal salts include sodium and potassium salts, particularly preferred are sodium salts. Alkaline earth metal salts include calcium and magnesium salts. Suitable alkyl groups include methyl, ethyl, propyl, butyl, pentyl and hexyl, preferred alkyl groups being methyl and ethyl. Where more than one alkyl group is present the groups may be the same or different. Preferred trialkylammonium salts are trimethylammonium salts and triethylammonium salts.

The salts of the invention are preferably in greater than 1 percent purity.

As 5α-pregn-16-en-3β-ol-20-one 3-sulfate ester sodium salt is a minor component of PREMARIN (conjugated equine estrogens), this invention also provides 5α-pregn-16-en-3β-ol-20-one 3-sulfate ester sodium salt in greater than 1 percent purity.

This invention also provides a compound consisting essentially of 5α-pregn-16-en-3β-ol-20-one 3-sulfate ester sodium salt, and a compound consisting essentially of a pharmaceutically acceptable salt of 5α-pregn-16-en-3β-ol-20-one 3-sulfate ester.

The present invention further provides compositions comprising a pharmaceutically acceptable salt of 5α-pregn-16-en-3β-ol-20-one 3-sulfate ester. In particular it provides compositions comprising at least 1% of a pharmaceutically acceptable salt of 5α-pregn-16-en-3β-ol-20-one 3-sulfate ester. One aspect of the present invention provides compositions wherein the only progestational agent is a pharmaceutically acceptable salt of 5α-pregn-16-en-3β-ol-20-one 3-sulfate ester. Embodiments of the present invention include compositions wherein the only active compound is a pharmaceutically acceptable salt of 5α-pregn-16-en-3β-ol-20-one 3-sulfate ester. In these embodiments other excipients and carriers may be included but no further active materials are included.

This invention further provides a method of using 5α-pregn-16-en-3β-ol-20-one or a pharmaceutically acceptable salt of its 3-sulfate ester as a progestational agent.

The present invention also provides a process for the preparation of a pharmaceutically acceptable salt of 5α-pregn-16-en-3β-ol-20-one 3-sulfate ester which comprises:
a) converting 5α-pregn-16-en-3β-ol-20-one 3-sulfate ester to a pharmaceutically acceptable salt,
b) converting a pharmaceutically acceptable salt of 5α-pregn-16-en-3β-ol-20-one 3-sulfate ester to a different pharmaceutically acceptable salt of 5α-pregn-16-en-3β-ol-20-one 3-sulfate ester or
c) converting 5α-pregn-16-en-3β-ol-20-one to a pharmaceutically acceptable salt of 5α-pregn-16-en-3β-ol-20-one 3-sulfate ester.

5α-pregn-16-en-3β-ol-20-one 3-sulfate ester may be converted to a pharmaceutically acceptable salt by neutralising the acid with an appropriate base, e.g. with an alkali metal carbonate, an alkaline earth metal carbonate or a primary, secondary, tertiary or quaternary amine carbonate. Alkali metal or alkaline earth metal salts may be prepared by using the appropriate alkali metal hydride e.g. sodium hydride, potassium hydride or lithium hydride.

A pharmaceutically acceptable salt of 5α-pregn-16-en-3β-ol-20-one 3-sulfate ester may be converted to a different pharmaceutically acceptable salt of 5α-pregn-16-en-3β-ol-20-one 3-sulfate ester by displacement, by using an ion exchange resin or by double decomposition (metastasis). Displacement of a weak base with a stronger one may be utilised to convert, e.g. an amine salt to an alkali metal salt or an alkaline earth metal salt using an appropriate base, e.g. a hydroxide. For example a trialkylamine salt such as a triethylamine salt may be converted to an alkali metal salt such as a sodium salt by treating it with an alkali metal hydroxide such as aqueous sodium hydroxide. The displacement may be carried out using an ion exchange resin. Alternatively one salt may be converted to another by double decomposition, e.g. an alkaline earth metal salt such as the calcium salt may be replaced with an alkali metal salt. E.g. the calcium salt of 5α-pregn-16-en-3β-ol-20-one 3-sulfate ester may be dissolved in water followed by the addition of e.g. sodium carbonate. Insoluble calcium carbonate would then precipitate out to provide the sodium salt of 5α-pregn-16-en-3β-ol-20-one 3-sulfate ester.

A pharmaceutically acceptable salt of 5α-pregn-16-en-3β-ol-20-one 3-sulfate ester may be prepared by directly converting 5α-pregn-16-en-3β-ol-20-one to a pharmaceutically acceptable salt of 5α-pregn-16-en-3β-ol-20-one 3-sulfate ester. This may be performed by reacting it with the appropriate aminesulfurtrioxide complex, e.g. by reacting it with a trialkylaminesulfurtrioxide (such as triethylaminesulfurtrioxide complex) to provide the corresponding trialkylamine salt (such as the triethylamine salt). If desired the salt may then be converted to another salt of the invention as described above. Alkali metal or alkaline earth metal salts may be prepared by treating 5α-pregn-16-en-3β-ol-20-one with the appropriate alkali metal hydride e.g. sodium hydride, potassium hydride or lithium hydride to produce the corresponding alkoxide *in situ* and then adding a trialkylaminesulfurtrioxide (such as triethylaminesulfurtrioxide complex) to provide the corresponding trialkylamine salt (such as the triethylamine salt). If desired the salt may then be converted to another salt of the invention as described above.

The compounds of this invention can be prepared from readily available starting materials according to the processes in Scheme I, as shown for 5α-pregn-16-en-3β-ol-20-one 3-sulfate ester sodium salt.

For example, according to Scheme I, 5α-pregn-16-en-3β-ol-20-one (1) [P.L. Julian et al., J. Am. Chem. Soc. 72, 5145 (1950)] was treated with one or more equivalents of triethylamine:sulfur trioxide reagent in a suitable solvent such as tetrahydrofuran at room temperature to afford 5α-pregn-16-en-3β-ol-20-3-sulfate, triethylammonium salt (2a). The triethylammonium salt (2a) was treated with aqueous sodium hydroxide to afford 5α-pregn-16-en-3β-ol-20-one-3-sulfate, sodium salt (2b).

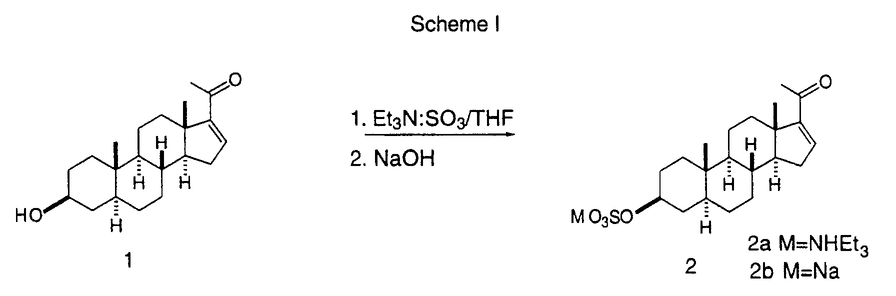

The present invention also provides pharmaceutically acceptable salts of 5α-pregn-16-en-3β-ol-20-one 3-sulfate ester prepared by a chemical process, particularly those prepared according to the processes described above. The invention also provides pharmaceutically acceptable salts of 5α-pregn-16-en-3β-ol-20-one 3-sulfate ester obtainable by such processes.

The compounds of this invention are progestational agents, and are therefore useful as oral contraceptives (male and female), in hormone replacement therapy (particularly when combined with an estrogen), in the treatment of endometriosis luteal phase defects, benign breast and prostatic diseases and prostatic and endometrial cancers. The compounds of this invention are also useful in protecting against epileptic seizures, in cognition enhancement, in treating Alzheimer's disease, dementias, vasomotor symtpoms related to menopause, and other central nervous system disorders The compounds of this invention are further useful in stimulating erythropoises.

The compounds of this invention can be used alone as a sole therapeutic agent or can be used in combination with other agents, such as other estrogens, progestins, or and androgens.

The compounds of this invention can be formulated neat or with a pharmaceutical carrier for administration, the proportion of which is determined by the solubility and chemical nature of the compound, chosen route of administration and standard pharmacological practice. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, lethicins, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compounds of this invention can also be administered orally either in liquid or solid composition form.

The compounds of this invention may be administered rectally or vaginally in the form of a conventional suppository. For administration by intranasal or intrabronchial inhalation or insufflation, the compounds of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermiable membrane covering a reservoir containing the active ingredient with or without a carrier, or a matrix containing the active ingredient. Other occlusive devices are known in the literature.

The dosage requirements vary with the particular compositions employed, the route of administration, the severity of the symptoms presented and the particular subject being treated. Based on the results obtained in the standard pharmacological test procedures, projected daily dosages of active compound would be 0.02 µg/kg - 750 µg/kg. Treatment will generally be initiated with small dosages less than the optimum dose of the compound. Thereafter the dosage is increased until the optimum effect under the circumstances is reached; precise dosages for oral, parenteral, nasal, or intrabronchial administration will be determined by the administering physician based on experience with the individual subject treated. Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form.

The following provides the preparation of representative compounds of this invention.

### Example 1

### 5α-Pregn-16-en-3β-ol-20-one-3-sulfate, triethylammonium salt (2a)

5α-Pregn-16-en-3β-ol-20-one (2.0g, 6.32 mmol) was dissolved in 50 ml of tetrahydrofuran. Sulfur trioxide triethylamine complex (1.2g, 6.6 mmol) was added. The mixture was stirred at room temperature for 20 hours. After addition of 50 ml of ether, the precipitate was collected on a Buchner funnel, washed with ether and dried; 2.4g (76%).
'H NMR (300Mhz, DMSO-d₆)
δ 0.78 (s, 3H), 0.80 (s, 3H), 1.19 (t, 9H), 2.21 (s, 3H), 3.10 (q, 6H), 3.92 (m, 1H), 6.87 (t, 1H), 8.80 (bs, 1H)
m/z (ES negative) 395(M-H)

### 5α-Pregn-16-en-3β-ol-20-one 3-sulfate ester sodium salt (2b)

Crude triethylammonium salt (2a) was stirred in IN sodium hydroxide at room temperature for 48 hours. The precipitate was collected on a Buchner funnel, washed with water and dried; 2.8g (85%).
¹H NMR (300Mhz, DMSO-d₆)
δ 0.78 (s, 3H), 0.80 (s, 3H), 2.20 (s, 3H), 3.92 (m, 1H), 6.87 (t, 1H)
m/z (ES negative) 395(M-H)

## Claims

1. A compound which is a pharmaceutically acceptable salt of 5α-pregn-16-en-3β-ol-20-one 3-sulfate ester.

2. The compound of claim 1 wherein the pharmaceutically acceptable salt of the 3-sulfate ester is an alkali metal salt, alkaline earth metal salt, ammonium salt, alkylammonium salts containing 1-6 carbon atoms or dialkylammonium salts containing 1-6 carbon atoms in each alkyl group, trialkylammonium salts containing 1-6 carbon atoms in each alkyl group and tetraalkylammonium salts containing 1-6 carbon atoms in each alkyl group.

3. 5α-Pregn-16-en-3β-ol-20-one 3-sulfate ester sodium salt, which is at least 1 percent pure.

4. A compound which consists essentially of a pharmaceutically acceptable salt of 5α-pregn-16-en-3β-ol-20-one 3-sulfate ester.

5. A compound which consists essentially of 5α-pregn-16-en-3β-ol-20-one 3-sulfate ester sodium salt.

6. A pharmaceutical composition which comprises a pharmaceutically acceptable salt of 5α-pregn-16-en-3β-ol-20-one 3-sulfate ester and a pharmaceutical carrier.

7. A compound as defined in any one of claims 1 to 5 for use as a medicament.

8. Use of a progestationally effective amount of a compound as claimed in any one of claims 1 to 5 for the preparation of a medicament for providing progestational therapy to a mammal in need thereof.

9. Use of an effective amount of a compound as claimed in any one of claims 1 to 5 for the preparation of a medicament for the treatment of cancers, central nervous system disorders, dementias, or Alzheimer's disease in a mammal in need thereof.

10. A composition comprising at least 1% of a compound as defined in any one of claims 1 to 5.

11. A process for the preparation of a pharmaceutically acceptable salt of a pharmaceutically acceptable salt of 5α-pregn-16-en-3β-ol-20-one 3-sulfate ester which comprises:
a) converting 5α-pregn-16-en-3β-ol-20-one 3-sulfate ester to a pharmaceutically acceptable salt,
b) converting a pharmaceutically acceptable salt of 5α-pregn-16-en-3β-ol-20-one 3-sulfate ester to a different pharmaceutically acceptable salt of 5α-pregn-16-en-3β-ol-20-one 3-sulfate ester or
c) converting 5α-pregn-16-en-3β-ol-20-one to a pharmaceutically acceptable salt of 5α-pregn-16-en-3β-ol-20-one 3-sulfate ester.

## Patentansprüche

1. Verbindung, welche ein pharmazeutisch akzeptables Salz von 5α-Pregn-16-en-3β-ol-20-on-3-sulfatester ist.

2. Verbindung nach Anspruch 1, worin das pharmazeutisch akzeptable Salz des 3-Sulfatesters ein Alkalimetallsalz, Erdalkalimetallsalz, Ammoniumsalz, Alkylammoniumsalze, die 1-6 Kohlenstoffatome enthalten oder Dialkylammoniumsalze, die 1-6 Kohlenstoffatome in jeder Alkylgruppe enthalten, Trialkylammoniumsalze, die 1-6 Kohlenstoffatome in jeder Alkylgruppe enthalten, und Tetraalkylammoniumsalze, die 1-6 Kohlenstoffatome in jeder Alkylgruppe enthalten, ist (sind).

3. 5α-Pregn-16-en-3β-ol-20-on-3-sulfatester-Natriumsalz, das mindestens 1% rein ist.

4. Verbindung, welche im Wesentlichen aus einem pharmazeutisch akzeptablen Salz von 5α-Pregn-16-en-3β-ol-20-on-3-sulfatester besteht.

5. Verbindung, welche im Wesentlichen aus 5α-Pregn-16-en-3β-ol-20-on-3-sulfatester-Natriumsalz besteht.

6. Pharmazeutische Zusammensetzung, die ein pharmazeutisch akzeptables Salz von 5α-Pregn-16-en-3β-ol-20-on-3-sulfatester und einen pharmazeutisch akzeptablen Träger umfasst.

7. Verbindung, wie in einem der Ansprüche 1 bis 5 definiert, zur Verwendung als Medikament.

8. Verwendung einer Progestations-artig wirksamen Menge einer Verbindung, wie in einem der Ansprüche 1 bis 5 beansprucht, zur Herstellung eines Medikaments zum Vorsehen einer Progestations-Therapie für einen Säuger, der deren bedarf.

9. Verwendung einer wirksamen Menge einer Verbindung, wie in einem der Ansprüche 1 bis 5 beansprucht, zur Herstellung eines Medikaments zur Behandlung von Krebserkrankungen, Erkrankungen des Zentralnervensystems, Demenzen oder Alzheimer-Krankheit bei einem Säuger, der deren bedarf.

10. Zusammensetzung, umfassend mindestens 1% einer Verbindung, wie in einem der Ansprüche 1 bis 5 definiert.

11. Verfahren zur Herstellung eines pharmazeutisch akzeptablen Salzes eines 5α-Pregn-16-en-3β-ol-20-on-3-sulfatesters, welches umfasst:
a) Überführen von 5α-Pregn-16-en-3β-ol-20-on-3-sulfatester in ein pharmazeutisch akzeptables Salz,
b) Überführen eines pharmazeutisch akzeptablen Salzes von 5α-Pregn-16-en-3β-ol-20-on-3-sulfatester in ein anderes pharmazeutisch akzeptables Salz von 5α-Pregn-16-en-3ß-ol-20-on-3-sulfatester, oder
c) Überführen von 5α-Pregn-16-en-3β-ol-20-on in ein pharmazeutisch akzeptables Salz von 5α-Pregn-16-en-3β-ol-20-on-3-sulfatester.

## Revendications

1. Composé qui est un sel pharmaceutiquement acceptable d'un 3-sulfate ester de 5α-prégn-16-èn-3β-ol-20-one.

2. Composé suivant la revendication 1, dans lequel le sel pharmaceutiquement acceptable du 3-sulfate ester est un sel de métal alcalin, un sel de métal alcalino-terreux, un sel d'ammonium, des sels d'alkylammonium contenant 1-6 atomes de carbone, ou des sels de dialkylammonium contenant 1-6 atomes de carbone dans chaque groupement alkyle, des sels de trialkylammonium contenant 1-6 atomes de carbone dans chaque groupement alkyle et des sels de tétraalkylammonium contenant 1-6 atomes de carbone dans chaque groupement alkyle.

3. Sel de sodium du 3-sulfate ester de 5α-prégn-16-èn-3β-ol-20-one, qui est au moins pur à 1 pour-cent.

4. Composé qui comprend essentiellement un sel pharmaceutiquement acceptable de 3-sulfate ester de 5α-prégn-16-èn-3β-ol-20-one.

5. Composé qui comprend essentiellement le sel de sodium du 3-sulfate ester de 5α-prégn-16-èn-3β-ol-20-one.

6. Composition pharmaceutique qui comprend un sel pharmaceutiquement acceptable de 3-sulfate ester de 5α-prégn-16-èn-3β-ol-20-one et un vecteur pharmaceutique.

7. Composé suivant l'une quelconque des revendications 1 à 5, pour une utilisation comme médicament.

8. Utilisation d'une quantité efficace sur le plan progestatif d'un composé suivant l'une quelconque des revendications 1 à 5, pour la préparation d'un médicament pour procurer une thérapie progestative à un mammifère en ayant besoin.

9. Utilisation d'une quantité efficace d'un composé suivant l'une quelconque des revendications 1 à 5, pour la préparation d'un médicament pour le traitement de cancers, de troubles du système nerveux central, de démences ou de la maladie d'Alzheimer chez un mammifère en ayant besoin.

10. Composition comprenant au moins 1% d'un composé suivant l'une quelconque des revendications 1 à 5.

11. Procédé pour la préparation d'un sel pharmaceutiquement acceptable de 3-sulfate ester de 5α-prégn-16-èn-3β-ol-20-one qui comprend :
a) la conversion d'un 3-sulfate ester de 5α-prégn-16-èn-3β-ol-20-one en un sel pharmaceutiquement acceptable,
b) la conversion d'un sel pharmaceutiquement acceptable de 3-sulfate ester de 5α-prégn-16-èn-3β-ol-20-one en un sel pharmaceutiquement acceptable différent de 3-sulfate ester de 5α-prégn-16-èn-3β-ol-20-one ou
c) la conversion de la 5α-prégn-16-èn-3β-ol-20-one en un sel pharmaceutiquement acceptable de 3-sulfate ester de 5α-prégn-16-èn-3β-ol-20-one.
